(19) **Europäisches Patentamt**
**European Patent Office**
**Office européen des brevets**

(11) **EP 1 281 068 B1**

(12) **EUROPEAN PATENT SPECIFICATION**

(45) Date of publication and mention of the grant of the patent:
**26.03.2008 Bulletin 2008/13**

(51) Int Cl.:
*G01N 25/48* (2006.01)  *G01K 7/22* (2006.01)
*G01N 17/00* (2006.01)

(21) Application number: **01933483.8**

(22) Date of filing: **08.05.2001**

(86) International application number:
**PCT/BE2001/000081**

(87) International publication number:
**WO 2001/085901 (15.11.2001 Gazette 2001/46)**

(54) **MICROPHYSIOMETER**

MIKROPHYSIOMESSGERÄT

MICROPHYSIOMETRE

(84) Designated Contracting States:
**AT BE CH CY DE DK ES FI FR GB GR IE IT LI LU MC NL PT SE TR**

(30) Priority: **08.05.2000 US 202475 P**

(43) Date of publication of application:
**05.02.2003 Bulletin 2003/06**

(73) Proprietor: **TTP Labtech Limited Royston,**
**Hertfordshire SG8 6EE (GB)**

(72) Inventor: **VERHAEGEN, Katarina**
**B-3000 Leuven (BE)**

(74) Representative: **Bird, Ariane et al**
**Bird Goen & Co c.v.**
**Klein Dalenstraat 42A**
**3020 Winksele (BE)**

(56) References cited:
**EP-A- 0 542 422**  **EP-A- 0 921 391**
**WO-A-95/11755**  **US-A- 5 255 976**

• **MAYER G ET AL: "NANOTITERPLATES FOR SCREENING AND SYNTHESIS" BIOMETHODS, BIRKHAEUSER, BASEL, CH, 1999, pages 75-128, XP000911754 ISSN: 1018-6255**

**Description**

**Field of the invention**

The present invention is related to a device and method for calorimetric measurements, especially to a high throughput screening device for detecting energy changes.

**State of the art**

Calorimetry in general is a measurement principle that detects all processes that occur in a reaction vessel. Calorimetry has several advantages: calorimetry is the most general detection principle, since most processes, physical, chemical or biological are accompanied by changes in heat content. This can be useful in the analysis of very complex processes, because it is more likely that unknown phenomena will be discovered. Moreover, the change of temperature of a reaction volume is dependent upon the concentration of the reagents, not upon the absolute quantity, offering the possibility of miniaturisation. Additionally, calorimetric methods are not dependent upon the sample form (the sample can be solid, liquid, gaseous, or any combination thereof, and neither colour, optical transparency, nor absence of suspended matter are requirements). Calorimetric measurements are non-invasive (it is not necessary to disturb the biological system, for example, by radiation). It even allows on-line monitoring of living cells over a longer period of time to obtain kinetic data. It is non-destructive towards the sample (no need for fluorescent markers or fixation procedures). The state of the art are devices capable of measuring small temperature differences between relatively large reaction volumes (typically 0.12 ml to 100 ml). SETARAM (Caluire, France) is a provider of calorimetric devices. These products are relatively large batch systems, mainly intended for applications in the biochemical field. The ITC® (Isothermal Titration Calorimeter, MicroCal, LLC, Northampton, USA) has a cell volume of 1.3 ml (and a detection limit of 400 nW). The TAM® (Thermal Activity Monitor, Thermometric, Järfälla, Sweden) has a cell volume of 4 ml (and a detection limit of approximately 100 nW). These micro-calorimeter batch systems are non-compatible with high-throughput requirements due to the relatively large volumes, the apparatus' closed structure and long cycle times (typical cycle times are 2 to 3 hours).
Commercially available micro-calorimetric sensors have also been described, using thermopiles and thermistors (Xensor Integration, Delft, The Netherlands). A main disadvantage of these devices is that they are configured in such a way that the reference temperature is the temperature of the silicon border. This leads to problems concerning baseline stability and common rejection mode since no differential measurements are used. Additionally, these devices are not compatible with standard robotics used in high throughput screening since no receiving cones are integrated.
The Cell Monitoring system (CMS®) developed by B. Wolf (University of Rostock, Germany) is a recently developed multiparametric chip including a pH microelectrode, oxygen and temperature sensor, reference electrode, a transparent aperture and interdigitated electrodes (IDES) to detect cellular metabolism. It is a modular system in which different sensors are "glued" together, resulting in a relatively large measurement chamber volume. This can cause problems with regard to quantification.
Physiometers are instruments designed to measure the activity of living cells and are sometimes referred to as whole-cell biosensors. They are mainly used for investigating cellular components, like ion channels in the cell membrane and for determining the effect of chemicals, physical stimuli and radiation on the activity of cells or tissue. Besides their usefulness in the field of pharmaceutical research, toxicology, oncology, and environmental studies they also have important potential applications in industry as a screening tool in the development of drugs and agrochemicals.
US Patent No. 6046056 describes a method of screening a plurality of test compounds for an effect on a biological system. Microfabricated substrates having one substrate and at least two intersecting channels fabricated in this substrate are used. The intersecting channels have a cross-sectional dimension from 0.1 $\mu$ to 500$\mu$. The device further contains a source of test compound fluidly connected to a first channel and a source of at least one component of the biochemical system fluidly connected to the second channel. The apparatus further comprises a detection zone in the second channel.
US Patent No. 5104804 relates to a microflow-chamber which makes use of a porous microchamber. At least part of the chamber wall is constructed from a porous material so that fluids can enter in the microchamber and cells cannot escape. The microchamber serves as disposable device for placing cells in the microflow-chamber so that the properties of the cells can be measured.
The industrial physiometers known in the art however make use of optical detection principles, usually a CCD camera to detect fluorescence or chemiluminescence in the reaction vessel.
International Patent Application W0974573 discloses a device and method for performing high-throughput screening of physiological response of cells to biologically active compounds. The method is based on multicolour luminescence reading. In this method, the cells contain at least one luminescent reporter molecule. Fluorescence or chemiluminescence requires labelling of molecules with the disadvantages described above. Moreover, only 50 % of the activity changes can be visualised by this method, whereby information on cell signalling can be lost. Furthermore, fluorescent and chemiluminescent probes can be hazardous for the environment.

EP-A-0921391 discloses a device capable of performing a differential heat measurement between a sample and a reference reaction. The device uses a thermopile to measure the differences in heat release between two receiving zones. However, this device is not compatible with the standard research formats and cannot be miniaturised while maintaining sufficient isolation between the measuring receiving zones, leading to false results and poor precision. Mayer G. et al. disclose in "Nanotiterplates for screening and synthesis", Biomethods, Birkhaeuser, Basel, CH, 1999, pages 75-128, an overview of the technique of nanotiterplates in various materials. It is shown how to adapt nanotiterplates to special requirements. In particular calibration of nanotiterplates is mentioned, but no hint is given how to obtain this. EP-A-0542422 discloses a multi-well microtiter plate. The microtiter plate disclosed comprises a heat exchanger to cycle the liquid specimens through pre-determined temperature regimes.

## Aims of the invention

The present invention aims to provide a new microphysiometer device, which can be used in a high-throughput screening procedure which can measure a broad scope of chemical and physical interactions.

## Summary of the invention

The present invention comprises an array device for monitoring the effect of a physical or chemical stimulus on multiple small samples, said array device comprising on a supporting substrate at least two array elements that are separated from each other by an isolation zone, said isolation zone being formed by at least part of said supporting substrate and being arranged to thermally isolate said array elements, each array element comprising:

- A receiving zone for retaining a small sample, said receiving zone having a wall and being arranged to provide a contact between one of said multiple small samples and said physical or chemical stimulus, said receiving zone having a cross-section smaller than 10 mm,

A differential heat detection means arranged to perform a measurement of heat between said receiving zone and a reference,

characterised in that said receiving zone of each of the array elements comprises a calibration resistor for calibrating the measurement of heat, the calibration resistor being situated in the wall of the receiving zone.

In the case a neighbouring receiving zone is used as reference, said differential heat detection means is preferably a differential heat detection means arranged to measure differences in heat between both receiving zones.

The array device of the present invention is preferably dimensioned as a standard 96, 384, 1536 or 6144 well microtiterplate.

The array device of the present invention can comprise resistors in said receiving zones. This set-up allows for providing thermosetting and/or thermal calibration means for every receiving zone in the array device.

In the array of the present invention, said receiving zones can comprise a bottom surface and sidewalls. Preferably, said bottom surface comprises said supporting substrate. In a specific embodiment of the present invention, said bottom surface is perforated.

Further, in the array of the present invention, said isolation zone preferably comprises a bottom surface, formed by said supporting substrate, and an isolator comprised between the receiving zones. Said isolator is preferably air.

In the array of the present invention, said differential heat detection means is preferably selected from the group consisting of thermopile, thermistor, diode, IR detection means and CCD camera.

The receiving zones according to the present invention can further comprise a detection means selected from the group consisting of a light addressable potentiometric sensor, a pair of interdigitated electrodes, a field effect transistor, a diode, a reference electrode, and a working electrode.

Possibly, the array of the present invention comprises receiving zones which are chemically modified to physically contain said sample and said chemical or physical stimulus within said receiving zone.

Advantageously, the receiving zones of the array according to the present invention have a cross-section of between 10 $\mu$m and 9 mm.

The substrate may be any of the group consisting of silicon, silicon dioxide, silicon nitride, silicon oxynitride, polysilicon, porous silicon, plastic, polymer, rubber, PVC, biodegradable polymer, glass, quartz, ceramics, aluminium oxide, agar, biological material.

The receiving zone may be capable of handling volumes in the range of 0 ml to 100 ml, preferably in the range from 0 ml to 7 ml, more preferably in the range from 0 ml to 5ml, most preferably in the range from 0.1 nl to 1 ml.

According to embodiments of the invention, the receiving zone may be a microvessel placed on top of, or hanging above the substrate. The microvessel may be made of any of the group consisting of metal, steel, silicon, silicon oxide, silicon nitride, silicon oxynitride, polysilicon, porous silicon, plastic, polymer, rubber, PVC, biodegradable polymer, glass, quartz, ceramics, aluminium oxide, agar, biological material.

According to embodiments of the invention the substrate and/or receiving zones may comprise a membrane.

According to embodiments of the invention, the array device may furthermore comprise a lid covering it.

According to embodiments of the invention, the array device may furthermore comprise a supply means which has the same dimensions and layout as the array device, except for the differential heat detection means which is omitted.

Another aspect of the present invention is a calorimetric measuring method for measuring multiple samples, said method comprising the steps of :

- Providing an array device according to the invention,
- Providing said samples in said receiving zones,
- Providing a chemical or physical stimulus to said samples and,
- Measuring the heat released by said samples.

A further aspect of the present invention is the use of the device of the present invention for enzyme discovery.

A further aspect of the present invention is the use of the device of the present invention for drug identification.

Another aspect of the present invention is the use of the device of the present invention for agrochemical identification.

## Short description of the drawings

Figure 1 a and b are illustrations of the device according to the present invention.

Figure 2 shows two neighbouring receiving zones on an array device according to the present invention.

Figure 3 depicts a perforated membrane or sieve.

Figure 4 draws a suspended membrane.

Figure 5 is an illustration of supply means based on capillary forces.

Figure 6 a to c shows a preferred embodiment of the present invention.

Figure 7 describes an api-Zym chamber: each well refers to a specific enzyme. The color change is measured optically.

## Detailed description of the invention

The present invention discloses a device for monitoring the effect of a physical or chemical stimulus on a sample by measuring the heat generated. The sample can be biological material or non-biological material, depending on the application, as described below. Preferably, according tot the present invention, a plurality of devices are set up in an array, contained in an apparatus. The device comprises a substrate. This substrate comprises at least a first and a second surface, and at least two substantially identical receiving zones or reaction vessels which form a part of the substrate. The receiving zone is used for retaining the sample and for providing contact between the sample and the physical or chemical stimulus. The device further comprises at least one heat detection means, preferably integrated in the substrate, for monitoring changes in heat content or enthalpy generated by the sample upon being contacted by a stimulus. Said heat detection means is arranged to perform a measurement of heat between said receiving zone and a reference. Said reference can be an external reference or a neighbouring receiving zone. A heat detection means can be selected from the group consisting of thermopile, thermistor, diode, IR detection means and CCD camera. A differential heat detection means can be operatively associated with a first and a second receiving zone so that a differential measurement between the first receiving zone and the second receiving zone can be performed.

Preferably, the differential heat detection means is positioned in such a way that the distance between the differential heat detection means and the first receiving zone is substantially identical to the distance between the differential heat detection means and the second receiving zone. The isolation zone surrounding the first receiving zone and the isolation zone surrounding the second receiving zone of the same device are preferably in substantially thermal equilibrium.

The differential heat detection means performs a differential measurement of heat or enthalpy (also called calorimetric measurement) between the two receiving zones. A device comprising such differential heat detection means is also referred to as twin detectors. Twin detectors have two identical receiving zones, one for monitoring a reference reaction and the other for monitoring the test reaction. The reference reaction is selected to resemble the test reaction as closely as possible. The signal is thus purely differential and disturbances like condensation or evaporation are cancelled out.

Techniques used to fabricate this array can be, but are not limited to, molding or micro-electronic processing. Micro-electronic processing offers specific advantages such as cost reduction if mass production is envisioned. Further, extreme miniaturisation is possible, the degree of which is limited by e.g. lithography. A high degree of parallelism is possible, making high-throughput feasible. Moreover, the detection limit of the system can be decreased considerably because fluidics are integrated on the same chip as the sensing element, reducing the length of the thermal path from reaction to sensing site. In addition, the detection limit of the system can be decreased even more if pre-amplifiers are integrated on the chip.

*Array*

An "array" as used herein refers to a set-up of at least two devices, which are substantially identical. The distance between neighbouring receiving zones of each of the at least two devices is substantially identical. Said array has preferably the format of a standard microtitre plate, such as, but not limited to a 96-well, 384-well, or 1536-well microtitre plate (see table 1).

*Table 1. Illustration of titre plate formats and use.*

| Format (number of wells) | max. volumes ($\mu$l) | inter-well distance (mm) | miniaturisation factor m |
|---|---|---|---|
| 96 | 200 | 9 | 1 |
| 384 | 80 | 4.5 | 2 |
| 1536 | 10 | 2.25 | 4 |
| 6144 | - | 1.125 | 8 |

Alternatively, the design of the array can be customised for integration in any high-throughput screening system. This allows the use of the array in standard robotics used in e.g. drug screening. In a preferred embodiment of the invention, the array is a sensor-arrayed chip with the footprint of a standard 96-well titre plate, and thus compatible with pharmaceutical robotics for dispensing and titre plate handling. The distance between adjacent wells in this format is 9 mm. For formats derived from this reference, the inter-well distance is 9 mm divided by the miniaturisation factor. The miniaturisation factor is defined as

$$m = \sqrt{\frac{n\_wells}{96}}$$

with n_wells the number of wells.
According to the present invention, the array is made with techniques allowing the formation of very small receiving zones compatible with standard arrays used.

*Sample and Stimulus*

The "sample" as referred to in the context of the present invention can be biological material or non-biological material. Biological material can be, but is not limited to, a bio-molecule, any biological cell or cell-culture (in monolayer or suspension), a tissue (natural or artificially generated), an organ, part of an organ, a micro-organism or a group of micro-organisms, one or more small animal, one or more plants . More specifically the biological material can be genetically modified to display specific characteristics, such as, but not limited to, the expression of a molecule such as, for instance a receptor or ion channel. Thus, according to a preferred embodiment of the invention, the first or reference receiving zone of the device can comprise an unmodified cell or culture thereof, while the second receiving zone comprises the cell or a culture of these cells which have been modified to display a specific characteristic. By contacting the cells of both receiving zones with a stimulus it is possible to monitor the effect of the presence of this specific characteristic on the reaction of the cell to the stimulus.

The biological material or sample can be applied directly or can be applied in a medium. Medium can be understood as any material or substance suitable for containing biological material, preferably keeping said biological material in its physiological state. Thus medium can refer to substances such as but not limited to cell culture media, physiological buffers, etc.

Non-biological material can be, but is not limited hereto, polymers, catalysts, ...

A stimulus as used herein refers to a chemical, biological or physical signal that is applied to the sample in order to determine its effect on the sample.

It will be understood that the nature of the sample and the stimulus in the present invention will be determined by the purpose for which the apparatus of the present invention is used. Additionally, the present invention, by providing the possibility to measure extremely small differences in temperature in extremely small reaction volumes, in circumstances which are compatible with biological cells, allows the use of calorimetric measurements for interactions that previously could only be detected by other, more invasive methods. A more detailed review of the applications and corresponding samples and stimuli associated therewith is provided in the examples herein.

In a preferred embodiment of the invention the apparatus is applied in high-throughput drug screening, whereby the sample can be a biological cell, such as, but not limited to, a human or animal cell in culture, including a cancer cell, and the stimulus comprises a library of drug candidates.

It will be understood that, in the context of the present invention, it can be envisaged that the sample and the stimulus are interchangeable. For instance, if both sample and stimulus are chemical molecules, it is clear that the choice of sample and stimulus will depend on the design of the measurement.

## *Differential heat detection means*

For the purpose of this invention, the interaction between the sample and the physical or chemical stimulus can be called "event".

In case an event is created, different signals can be obtained such as change of heat or enthalpy, change of ionic concentrations of different ions, etc...

The differential heat detection means (DHDM) of the present invention is in contact with the two receiving zones at a first and second detection zone. For most applications one of the receiving zones will be used as a reference while in the other an event is generated. When a change of heat or enthalpy is generated, this signal is measured by the DHDM, whereby the thermal input signal is converted into a differential electrical output signal. For instance, to test the effect of a chemical on a biological cell, both the first (reference) and second receiving zone will contain the biological cells. The reference sample will be contacted with a placebo solution, while the other is contacted with the chemical. Alternatively, such as in e.g. drug screening applications, reference cells are present in a first receiving zone and genetically modified cells are present in a second receiving zone. When e.g. a chemical stimulus is added to both receiving zones, a difference in effect on the genetically modified cells will cause a heat change at one side of the differential heat detection means, thereby producing a differential voltage. Preferably, the reference sample is thus substantially equivalent to the test sample, so that heat capacities and surface relationships do not influence the measurement.

The device as such has a very high common mode rejection ratio, offering a signal which originates from the chemical or physical stimulus stimulating or suppressing the metabolism of the cells under study. The isolation between the first receiving zone and second receiving zone should be high, so that the processes in both receiving zones do not influence each other, e.g. the temperature in one receiving zone is preferably not influenced by the temperature in the second receiving zone. This is accomplished by ensuring an isolation zone around the receiving zones (see "receiving zone" below) and around each device. The thermal isolation of the isolation zone between two neighbouring devices ("inter-device isolation zone") is at least equivalent to that of the isolation zone between the two detection zones within a device. Preferably, the thermal isolation between the devices is higher than the thermal isolation between each of the receiving zones of the same device.

According to a preferred embodiment of the invention, the differential heat detection means is a thermopile, consisting of a set of 2 temperature sensitive means with a differential read out. Said thermopile has a hot and a cold junction, which are operatively associated with the first and the second receiving zone respectively. Said hot and cold junction are thermally isolated one from another.

A thermopile is a sensor that inherently makes use of the twin arrangement principle. A thermopile is made up of a number of thermocouples, electrically connected in series, thermally connected in parallel.

The main advantages of a thermopile are:

- The thermopile is a self-generating offset-less device, as the heat flowing through it supplies the power for the output signal. As a result there is no offset drift and no interference caused by power supplies.
- The sensitivity of the thermopile is hardly influenced by variations in the electrical parameters across the wafer or by the temperature.

The thermopile can be optimised in terms of dimension and number of thermo-electric strips. The optimum is found analytically, with the help of MATLAB. The other sensing principles and supporting structures (e.g. the resistors in the capillary walls) are designed to fit in this optimised thermopile chip architecture

## *Substrate*

The substrate as used in the context of the present invention can be any material that allows the fabrication of the array device in the dimensions of a microtiterplate. The substrate can be, but is not limited to, one of the group consisting of silicon, silicon oxide, silicon nitride, silicon oxynitride, polysilicon, porous silicon, plastic, polymer (also rubber, PVC, etc...), biodegradable polymer, glass, quartz, ceramics, aluminium oxide, agar, biological material, and rubber. When the substrate is a material compatible with semiconducting processing, the integration of sensors and fluidics on the same chip is facilitated. This opens the way to small sample volumes and inert reaction vessels, since no reaction with materials or adsorption of materials occurs.

## *Receiving zone*

For the purpose of this invention, the term receiving zone or reaction vessel is used to refer to a means or carrier of the sample. More specifically, if the sample used is biological material, such as a cell culture, the receiving zone will preferably be of a format capable of containing fluids. Preferably, said receiving zone is capable of handling volumes in the range from 0 ml to 100 ml, more preferably from 0 ml to 7 ml, especially preferably from 0 ml to 5 ml, most preferably from 0.1 nl to 1 ml. Preferably, said receiving zone has a maximum volume of 5 ml.

The receiving zone should operatively be associated with the differential heat detection means. This means that there is a thermal coupling between the receiving zone and the temperature sensitive part of the differential heat detection means. This can be, but is not limited to, the hot or cold junction of the thermopile.

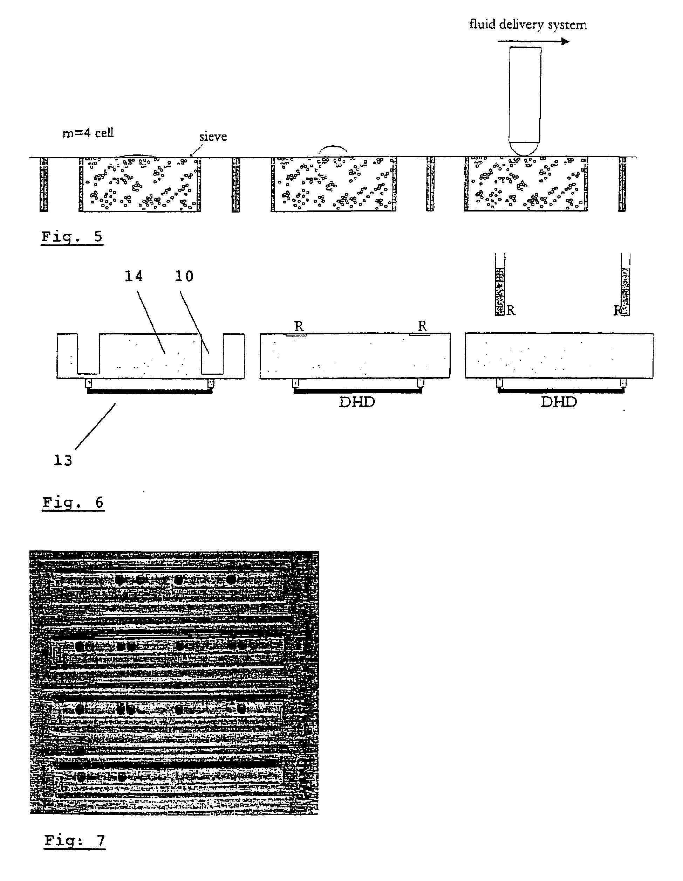

According to a preferred embodiment of the invention, the receiving zone is formed by a recess (10) in a substrate (see figure 6)). The recess can have any 3-dimensional shapes and can be, but is not limited to, cylindrical shaped, cubic shaped, bar shaped, truncated pyramidal shaped, truncated conic shaped. Said recess can extend from the first surface of the substrate to the second surface or can extend from the first surface tc the bulk substrate material. Said receiving zone can also be an area on the substrate e.g., the whole substrate or parts of the area of the substrate can be chemically or physically modified in such a way that they can selectively hold one or more of said sample, medium, and chemical stimulus.

Alternatively, the receiving zone can be a microvessel made of a material, such as but not limited to, metal, steel, silicon, silicon oxide, silicon nitride, silicon oxynitride, polysilicon, porous silicon, plastic, polymer (also rubber, PVC, etc...), biodegradable polymer, glass, quartz, ceramics, aluminium oxide, agar, biological material, and rubber, which is either placed on top of, or is hanging above the substrate. For instance, the receiving zone can be formed by a needle form dispenser. Preferably, the distance between the substrate and the receiving zone is not larger than the distance between two adjacent receiving zones In Figure 6, the differential heat detection means is referred to as 13, the substrate is referred to as 14.

Said receiving zone is surrounded by an "isolation zone", which functions as a thermal isolation zone.

## *Membrane*

A membrane as used herein refers to a part of the device which provides thermal isolation. The membrane can be part of the substrate or can be a second substrate. When the receiving zones are formed by a recess in said substrate, a membrane can cover the recess at either the first surface or at the second surface of the substrate. When the recess extends from the first surface of the substrate to the bulk material, and the substrate from the bottom of the recess (the wall of the recess being substantially parallel with the first or the second surface of the substrate) to the second surface forms an insulating layer, this part of the substrate is considered to be the membrane.

When the receiving zone is formed by complete etches through the substrate extending from the first surface to the second surface, a thin membrane can be formed for covering said recess at a first side or at the second side of the substrate.

The membrane can be made of the substrate material or of another material. The membrane can be formed, by methods such as, but not limited to, growing a membrane layer on the substrate cr bonding a membrane.

The membrane can also be made of another material than the substrate material. For instance, a thin layer of the membrane material can be formed on the substrate material. The recess can be formed in the substrate by conventional micromachining techniques. The recess can extend from the first surface of the substrate until the membrane. When e.g. dry etching techniques are used, the membrane material and etch chemistry can be chosen such that a recess is etched in the substrate and that the etching process selectively stops on the membrane. The thickness of the membrane can be between 1 um and 1 cm, preferably between $1 \mu m$ and 1 mm, most preferably between $10 \mu m$ and 0.1 mm. The thickness depends on the membrane material, and consequently on the thermal isolation of the membrane material. For example, the thickness of a silicon oxide membrane can be lower than $10 \mu m$. For glass, the thickness can be between 1 and 5 mm.

Any of the above mentioned materials can be perforated and/or patterned material such that a perforated membrane or sieve is created. When a liquid is supplied to the receiving zone, oxygen molecules can escape through these openings. Additionally or alternatively, the sieve serves to encapsulate biological material. Sieves are useful when specific elements, like cells or tissue, must be entrapped inside the physiometer, while maintaining (periodic) contact with a nutritive medium.

The sieve's square openings are made in a membrane in such a way that that their width is the same as the width of the surrounding bar. The size of the openings is determined by the function of the sieve. For instance, when desiring cellular entrapment, the openings will preferably have a diameter of about 6 $\mu$m, while for tissue entrapment the openings will preferably have a diameter of about 20$\mu$m. It is understood that the size of the openings can be optimised depending on the sample used.

The sieve is preferably made of low stress material (1.2 $\mu$m plasma oxide). In places where sensors are positioned, other structural layers are present.

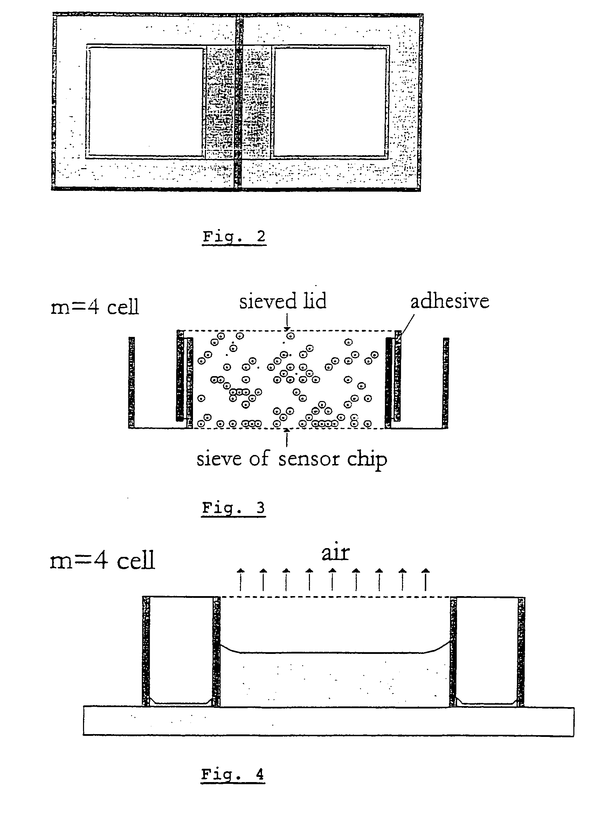

When the receiving zone is a recess in the substrate, the openings will extend from the bottom of the recess to the second surface of the substrate. When the receiving zone is part of the first surface, the openings will extend from this first surface to the second surface. Said membrane can also be a suspended membrane (see figure 4).

The device or array of devices can further comprise a membrane which covers it. This membrane can also be called "lid". The lid can be made of at least one material such as, but not limited to, metal, steel, silicon, silicon oxide, silicon nitride, silicon oxynitride, polysilicon, porous silicon, plastic, polymer (also rubber, PVC, etc...), biodegradable polymer, glass, quartz, ceramics, aluminium oxide, agar, biological material, and rubber.

The same sieve structure as described for the membrane can be found in the lid. The lid can cover the receiving zone, as shown in figure 3. In this way, cell or tissue can be entrapped.

*Extra detection means*

According to one embodiment of the invention, the device can further comprise an extra detection means. Said extra detection means can be a light addressable potentiometric sensor (such as, but not limited to, a Light Adressable Potentiometric sensor or "LAPS"), a FET device, a diode, (interdigitated, "IDES") electrodes, a reference electrode, a working electrode and/or an impedance spectroscopic device. Said extra detection means can be placed such that it is operatively associated with said receiving zone. For example, said extra detection means can be integrated in the substrate. When the receiving zone is a recess in the substrate, the extra detection means can be integrated in e.g., the side walls or the bottom wall of the recess When the receiving zone is an area on the substrate, the extra detection means is preferably integrated in said substrate. The number of extra detection means per receiving zone is not limited.

*Calibration means*

In the context of the present invention it is envisaged that, in particular circumstances or for certain applications it may be required to set the device at a given temperature. Therefore, the device may further comprise a calibration means for thermosetting said device. The calibration means can be a tuneable electrical power generating means such as e.g. a resistor or a thermopile or a temperature sensitive means such as a resistor, diode, thermopile or a microprocessor that drives the power generator in such a way that a pre-determined temperature is obtained.

*Supply means*

Additionally, the device of the invention can comprise a supply means to fill the receiving zones in an active (e.g. pressure or suction force) or passive (e.g. capillary force) manner. Said supply means can be fabricated in a material such as, but not limited to silicon, silicon oxide, silicon nitride, silicon oxynitride, polysilicon, porous silicon, plastic, polymer (also rubber, PVC, etc...), biodegradable polymer, glass, quartz, ceramics, aluminium oxide, agar, biological material, and rubber. In a preferred embodiment of the invention, the supply means has the same dimensions and layout as the device, except the differential heat detection means is omitted. The supply means can optionally have external or internal (e.g. made by micromachining) pumps and valves. Alternatively, said supply means can also be an industrial dispenser with or without external pumps and valves. The supply means can also be a custom-made dispenser with or without external or internal (e.g. made by micromachining) pumps and valves.

In the preferred embodiment, the array makes use of capillary forces to supply the solutions to the receiving zone(s). Figure 4 illustrates one way to accomplish this. The sample solution only fills those receiving zones which are either open-ended or sieve-ended, because the air must be able to escape. A hydrophilic native silicon oxide covers the inner walls of the capillaries. To avoid wetting of the whole chip via the membrane, hydrophobic polyimide rings are deposited on the bottom of the rims of the capillary.

The height *h* of the fluid column inside the a capillary is:

$$h = \frac{2\gamma}{\rho g r} \cos \Phi$$

with $\gamma$ the surface tension of the analyte solution (72 mN/m$^2$ for water), $\rho$ its density, g gravitational acceleration, r the equivalent hydraulic radius, and $\phi$ the contact angle between the analyte and the capillary. If water is applied in the capillary of the 384-well micro titre plate (m=2) the height equals 4.1 mm ($\phi$=60°), 6.8 mm ($\phi$=35°). The water column will thus fill the whole capillary. If different solutions need to be tested on the chip, commercial dispensing tools can be used up to the m=4 case. For higher density chips, piezo-driven dispensers are needed.

In high-throughput drug screening, the potential drug candidate is diluted in water or dimethyl sulfoxide (DMSO). One possible way to accomplish this is illustrated in Figure 5. In a first phase, the chip is filled with water or DMSO, by using capillary forces. In a second phase, a dispensing tool supplies the drug candidate. Diffusion processes (passive) accomplish mixing.

The emptying is accomplished by contacting the meniscus formed due to gravity, with a hydrophilic surface. Washing of the capillaries can be accomplished by repeating the passive filling and emptying several times.

The device of the present invention can further comprise read-out electronics such as (pre-) amplifying, multiplexing, filtering and/or buffering circuitry.

For calibrating said the calorimetric measurement, resistors, other than those used for thermosetting, are used as calibration means and are situated in the walls of the recesses (1 recess - 1 calibration resistor). The calibration resistor is designed to be able to produce dissipation heat in the same range as the produced biological power. Its value is preferably 800 k .

## Examples :

### Example 1: Development of a microphysiometer comprising a thermopile as differential detection means.

A microphysiometer was developed, which comprises a device which allows the defection of extremely small temperature differences between two reaction vials, which is biocompatible and of which an array can be formatted which has the footprint of a microtitreplate. In order to correspond to a 96-well plate format, the distance between the centre of the two substantially identical receiving zones of the device was designed to be 9 mm.

As substrate material for the device silicon was selected.

The herein described device was developed for processing on an 8 mm membranous chip. However, it will be understood that, the use of other substrate material may dictate adjustments in certain described settings.

The use of silicon as substrate material provides the possibility of integrating the sensors and fluidics on the same chip, so that sample volumes can be minimalized (minimal dead volume). Additionally, by forming the receiving zones as recesses in the substrate, inert receiving zones are obtained, as no reaction or sorption of materials occurs. Because of its good thermal conductivity, making thermal sensors of silicon requires specific measures. The bulk material short circuits the thermal signals, and should be removed by post-processing.

As differential heat detection means a thermopile was integrated in the substrate. Additionally, 2 resistors were integrated in the walls of the recesses forming the receiving zones as thermosetting means.

As described above, the device comprises, besides the thermopile, two receiving zones and two resistors. Each of the receiving zones is surrounded by an isolation zone.

The receiving zones are formed by providing a recess in a substrate and can be seen as vertical capillaries extending from the first surface of the substrate to the second surface of the substrate. Thus, the receiving zone is a hollow silicon volume, in substantially upright position in respect to the wafer plane. They have square cross-sections and they are dry-etched in the silicon substrate. A thin layer of a membrane material (different from the substrate material, substrate=silicon; membrane=silicon dioxide) is formed on the second substrate or membrane material, the etching process will stop or the silicon oxide. Due to the dry etching process, the corners of the square are rounded. The height of the recesses is substantially the substrate thickness. In case of a 6 inch wafer, this is 625 $\mu$m, in case of an 8-inch wafer, 725 $\mu$m. If necessary, the substrate can be made thinner.

The use of vertical capillaries has many advantages over droplet dispensing:

(i) It increases the volume to contacting surface ratio of the analyte solution to the chip. This results in a more compact chip for the same sample volumes,

(ii) it decreases the evaporation rate. The free contact surface of the sample is only at both open edges of the receiving zone.

The membrane was perforated to obtain a sieve of which the openings were chosen to be 6 μm wide, to enable cellular entrapment.

Each of the receiving zones of the device was provided with a thermal isolation zone which corresponds to a recess in the substrate. Each receiving zone is bordered by a double silicon rim, which encloses the thermal isolation zone so that the inner rim is common between the thermal isolation zone and the receiving zone. The width cf the rims must be minimal to maximise the thermal isolation between two neighbouring receiving zones. The minimal dimensions are set by:

(i) mechanical stability issues: in order to give decent support to the membranous chip, the minimal rim width between two neighbouring devices, is set to be 100μm, meaning 50μm for each receiving zone + accompanying isolation zone,
(ii) (ii) technological issues: dry etching technology easily allows for etch ratios of 15. For processing on an 8-inch wafer, with a thickness of 725μm, a rim width of 50 μm, which is feasible and not critical, was chosen.

A device comprises two receiving zones and two isolation zone. The units of 1 receiving zone and one isolation zone are mirrored around an Y-axis.

The devices can also comprise a thermosetting means. Two resistors are integrated in the walls (surrounding rims) of each receiving zone, one of which is the calibration resistor, is the other the thermosetting resistor. The latter is designed to be able to produce dissipation heat to heat up the receiving zone to a temperature in the range of room temperature to 100°C in 1 min when filled with water. Its value is 130 k .

Reference and working electrodes are present in each recess.

**Example 2: development of different arrays of the devices**

The device as developed in Example 1 was designed for a sensor-arrayed chip with the footprint of a standard 96-well titre plate, to be compatible with pharmaceutical robotics for dispensing and titre plate handling (the 96-well format of microtitre plates being considered as the reference for the moment). The distance between adjacent wells in this format is 9mm. For formats derived from this reference, the inter-well distance is 9mm divided by the miniaturisation factor. The miniaturisation factor is defined as:

$$m = \sqrt{\frac{n\_wells}{96}}$$

with n_wells the number of wells. The format used in the present example is a 1536-well (m=4), with 8*(8*12) devices on the substrate.

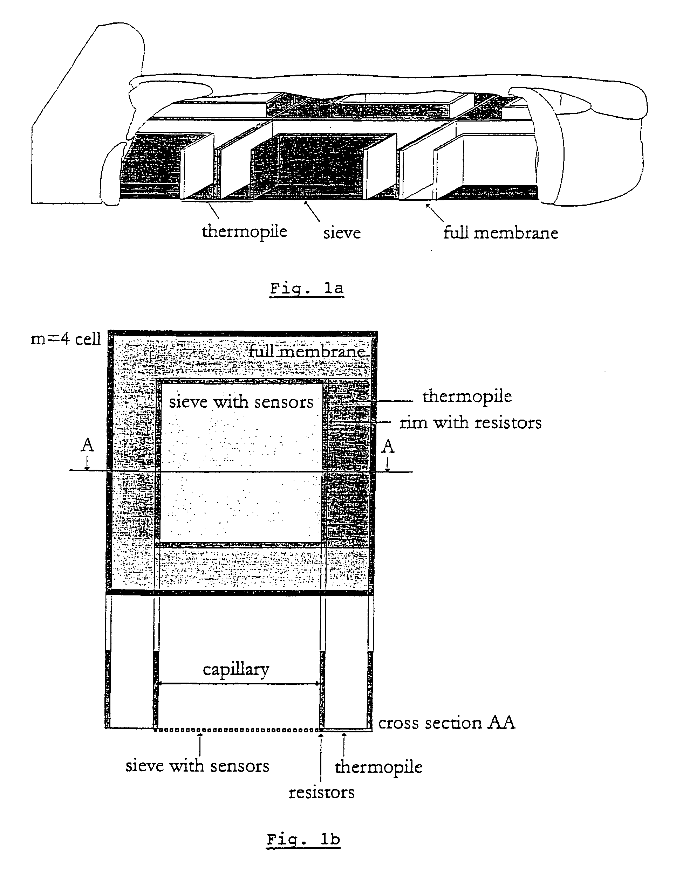

A 3-D view of a part of the array is shown in figure 1a. A thermopile connects each two neighbouring cells.

**Example 3: Applications of the microphysiometer of the present invention.**

The device of the present invention makes it possible to measure the interaction between two molecules in a very sensitive, physiologically relevant way, while its scale allows integration into industrial robotics, opening enormous possibilities for its use as a high-throughput screening tool.

A. Industrial enzyme discovery

According to a preferred embodiment of the invention the device is used for screening enzyme activity in the identification of new enzymes.

The field of industrial enzyme discovery is now profiting from the genomics and assay technology revolution. Analysis of gene expression libraries from various organisms allows the discovery of many enzymes, or biocatalysts, which brings new solutions to industry. The use of enzymes to effect chemical transformations, also called biocatalysis, has grown enormously over the last 15 years, and is now a major contributor to industrial synthesis of fine chemicals and pharmaceutical precursors.

Traditionally, cultures of different bacteria are set up and cell extracts are prepared. These are then tested for active components, which are purified. The corresponding DNA sequences is isolated and expressed in a domesticated host in the hope the same active component can be obtained. The use of gene expression libraries has brought significant advantages: it avoids having to keep varieties of bacteria cultures, many of which require specific conditions (i.e. thermophyles); and it allows rapid identification of the active molecules and the DNA sequences encoding them. Furthermore, expression can be directly tested in the desired domesticated host. However, the bottleneck in the identification of new biocatalysts remains the screening of the cell extracts and expression libraries for the production of enzymatic activity on the target of interest. Often a high number of extracts or expression products are generated and a method is needed to allow sensitive and rapid identification of the clones/extracts that produce/contain molecules that are active on the target, and this on a large scale. The following describes how such a screening can be performed with the device of the present invention.

1. Preparation of the sample

a) bacterial cell culture: a collection of different bacteria strain is grown at respective optimal temperatures; a cell extract of each strain is prepared by disrupting bacteria with either ultrasounds or a lysis buffer. All extracts are normalized at 1 mg/ml and are distributed 96 or 314 well in a registered order.

b) an enzyme library can be purchased from suppliers or prepared from bacteria containing a plasmid from which an enzyme can be expressed. Such library can be prepared in a microplate format. The enzyme concentration is then normalized to a specific concentration which is to be determined in each case. The libraries can contain many unique enzyme variants with yet unidentified properties. The different enzymes can be tested for the improvement of various industrial processes.

Plates can be stored at -80°C or -20°C for several weeks.

2. Screening operation

If an enzyme that modifies target A (i.e. A is the "substrate" in chemical terms) is to be found, target A is prepared in a screening buffer and constitutes the sample.

- A number of reference receiving zones (blanks) are loaded with screening buffer, while the reaction receiving zones are loaded with target A in screening buffer. The temperature is monitored in real time.
- The reaction receiving zones are then loaded with the extracts or cell supernatants (or (partially purified fractions thereof) containing the enzyme. As a control, an enzyme known to act on target A is added in a number of reaction receiving zones (instead of the extract or supernatant). Preferably the solution of control enzyme is in all aspects (except for the presence of the control enzyme) similar to the extract or cell supernatant solutions tested. Temperature is monitored in real time.

If the extracts or supernatants contain enzymatic activity which act on the target in the sample, the reaction enthalpy will heat up the reaction receiving zone and this will be detected by the DPDM. Additionally, if a pH detector is also included in the device, the possible accompanying change in proton concentration could be detected by means of the pH detector. Enzyme potency is function of the quantification of temperature change in time. Any cell extract that generates heat relative to the control wells is considered a hit. The well coordinates will allow the identification of the corresponding bacteria strain or clone.

If the organisms are "extremophyles" which grow at very high temperatures (70 to 100°C or above), the enzymatic activity can still be monitored at such temperatures using the thermosetting unit.

3. Processing of the hits

In the case of the screening of bacterial extracts, the "hit" extract can be further fractionated to determine, optionally, with the same device, the sub-fraction localization of the enzyme. Such fractions containing the enriched enzyme can then be used as biocatalyst preparation for industrial purposes.

The expression genome of the studied bacterial strain can then be prepared as a cDNA library and further subcloned in an expression vector. The library can then be introduced in a suitable host (Phage, E. coli, B. subtilis, etc...) and induced. Clones containing a single copy of each gene can then be isolated and grown in a microplate format, while keeping a reference microplate for each screening plate. Cell extracts can then be prepared and screened for the desired activity as described earlier. A hit well will then correspond to a clone in the reference microplate. The plasmid containing the desired gene can then be amplified, sequenced and isolated for further processes such as bulk enzyme production. Such preparations can be used for industrial purposes.

In the case of direct screening of cDNA libraries the "hit" can be directly identified as a desired compound. The enzyme can be identified and purchased or prepared using the corresponding "hit" clone to obtain quantities suitable for industrial processes.

B. Drug Identification and development

The present invention provides a screening tool for the easy identification of interaction between a ligand and a target, opening up important perspectives in drug screening.

1. Measurement of the biological profile of compound libraries at high throughput

High throughput screening of compound libraries is a major step in the process of drug discovery. High throughput screening is often also required during the lead optimization phase as well as for drug development. Thus the object of the screening can either be to screen several thousands or even millions of compounds for their pharmacological activity on a target or to test the toxic activity of a selected compound on a number of non-targets. Pharmaceutical, biotech or agro-chemical companies have compiled collections of compounds from various sources in order to discover molecules that can be developed into potential drugs or agrochemicals.

Both pharmacological and toxic activities are the result of the interaction between compounds and biological components of the cellular machinery. Such interaction process is usually related to the binding of the compound to receptors, enzymes, DNA, structural proteins, membranes or any other biological molecule or structure. The intervention in the normal regulation of cellular processes will induce one or more metabolic or structural modifications, which can be detected by measuring cellular enthalpy changes.

Screening compound libraries ranging from 50000 to several millions of molecular entities requires a heavy investment in machinery, reagents and data analysis and usually ends up with the selection of only a few molecules.

Whereas coverage of the "chemical space" has been widely discussed and worked out in the design and selection of compound libraries, the "biological space" is rarely foreseen. Lipinsky (reference?)has recently published the analysis of several hundreds of drugs and has come up with a list of five molecular parameters which may be required. Although this rule of five is now applied in the selection and synthesis of compounds it does not guarantee the potential of such molecules to become drugs nor to exhibit biological activities. Indeed the compound selection according to these simple rules could even reduce the chances of discovery.

It is an object of the present invention to detect the changes in cellular enthalpy (or of the surrounding environment) caused by the activity of the molecule on the target as a temperature change using microcalorimetry. Moreover, the present invention makes it possible to perform such measurements in a high-throughput set-up. Additionally, by integrating pH metry in the device, the effect on the ionic balance can be recorded. In this way the "biological space" of any compound collection can be effectively assessed.

Such method can refine any compound library. Screening only the biologically active molecules dramatically increase the probability to find pharmacologically active molecules. It will also reduce notably the amount of compounds to be screened.

Different strategies for drug screening can be envisaged in the context of the present invention, depending on whether or not the target is well-defined. Preferably, a library of compounds is tested on the target. If the target is a known enzyme, this enzyme can be used either as the "sample" or the "stimulus" in the screening procedure. Alternatively, it can be envisaged that cell-cultures (unmodified or modified to express specific target molecules) can be used as sample to screen for a ligand which binds (and possibly also influence the activity of) the target. Possibly, the target is a protein of which the function is still unknown, and identification of a specific ligand can help assess the target function.

Large compound collections can be made starting from natural small ligands. The molecule classes can comprise: amino acids (naturals and derivatives), carbohydrates (glucose, fructose, sucrose, etc...), lipids (fatty acids, Prostaglandins, leukotrienes, etc...) nucleotides (NTPs, NDPs, etc...), neurotransmitters (GABfi., Serotonine, Dopamine, Glutamate, etc...), small peptides (NPY, vasopressin, etc...), enzyme co-factors (e.g. NAD, FAD, NADPH, acetyl-CoA, etc...), and others.

If the target is a known molecule it can be produced in a purified form and in bulk quantities. Screening plates are prepared by replication/dilution of stock plates of the compound library and addition in duplicate in a microtiterplate for screening. Control wells contain a known ligand of the target. Upon addition of the target, heat change is monitored. An interaction between the target and a ligand will be detected by heat change; the corresponding ligand molecule is called a hit.

Alternatively, the library of compounds can be tested on one or more cell types. Cultures of these cells are grown in an array of receiving zones of the device. The compounds of the potential drug library are applied as stimulus. In control receiving zones, a known stimulus can be added. When working with genetically modified cells as target samples, the control will be the non-modified cell-line. If a compound is active on the cell-line tested (binding to membrane, modifying membrane structure, entering the cell, disrupting cellular components, binding or interaction with intracellular molecules etc.) or in any way modifies the physiological state of the cell this will be measured as a temperature change in-or outside the cell. A library of known biologically active molecules (such as Tocris or RBI) can be used as a reference and to differentiate active molecules from the others.

The corresponding molecule is selected as a hit and can easily be identified by its coordinates in the microtitre plate. Active molecules are labeled as positives in the related database; such hits can then be "picked" from the stock plates and re-distributed in new stock plates. This process can be done using automated equipment. The newly formated library can be called "enriched library" and commercialized or screened for more specific targets

C. Identification of Agrochemicals

As mentioned above, the device of the present invention can also be of interest in the screening of compounds in the identification of agrochemicals, particularly in the search for novel herbicides, pesticides and fungicides. Envisaged targets can be either plant, animal or fungal cells, respectively or can be specific target molecules which are known to be crucial for the normal function and development of these cells (such as DNA, RNA, enzymes involved in the synthesis of amino acids, or proteins etc.). Libraries of compounds can be screened for their activity and the hits can be further developed into active compounds. Alternatively, the device of the invention can be used to screen libraries of structural variations of a known compound that has been slightly modified in order to increase/decrease its specific or nonspecific (undesired) activity in the improvement of known chemicals. This can result in a significant reduction in time and effort needed compared to classical synthesis and testing approaches.

D. Detection and Identification of bacteria

Microbial contamination concerns are well documented and this problem is gaining momentum as more outbreaks caused by contaminated water or food are reported and greater populations are affected. The main bacteria strains are E coli, Listeria, and Salmonella. Economic losses to the food production industry are significant as bacterial contamination shuts down processing and scours the reputation of businesses. To date, traditional culture plate methods have been used to monitor food production batches. These methods are labour intensive and time consuming and, most importantly, require incubation times of 24-72 hours. The need for an easy, fast, accurate and sensitive bacterial detection method is immediate and crucial to maintaining public health.

Moreover, the most sensitive assays to detect bacterial strains are based on ELISA which are time consuming and rather expensive. The API-ZYM™ system from biomerieux uses enzymatic profiling as a tool to not only detect bacteria but also provide detailed information about strains and/or species. The system is based on strain specific features concerning enzyme expression. Thus a combination of enzyme expression is designed to detect one bacteria specie. This test is usually performed on a barrette (see figure 7) where a combination of 19 enzymes allows rapid identification of bacteria. A similar system is used in Biolog MicroPlates™. These are standard 96-well microplates that are pre-loaded with metabolic tests for characterizing and/or identifying microbes. Each well of the microplate contains a different carbon source along with redox chemistry in a dry form. The chemistry is rehydrated by inoculation with a cell suspension, a procedure that takes less than 1 minute. Utilization of the carbon source in a well results in a redox reaction, which forms a purple color or a turbidity change.

Again, culture is required and sensitivity is an issue.

Microcalorimetry appears then as the method of choice to quickly and accurately detect enzymatic activity from low amounts of bacteria.

*Materials:*

Test samples:

Sterile cotton swabs are used to swipe the material to be screened (food, hands, surfaces, etc...), they are incubated in 15 ml tubes with 10 ml of incubation buffers; incubated under shaking for 15 minutes. The cotton swab is then removed and tubes are centrifuged at 20 000 rpm for 15 minutes to pellet all organisms. The pellet is resuspended in 100 $\mu$l of incubation buffer to prepare the test sample.

Screening plate:

The test plate is prepared as follows:

A set of 19 specific enzyme substrates is diluted at a concentration of 5 $\mu$M and distributed in test wells of the microcalorimetry microplate; whereas reference wells contain an equivalent volume of incubation buffer.

*Procedure:*

Microcalorimetry is monitored in real time in the test plate; 2 to 5 $\mu$l of the test sample is then added in all duplicate wells. The heat change generated from control wells is then registered. If any enzyme present in the test sample in a specific well reacts with a specific substrate; the change in heat will be detected within the microcalplate. Enzyme potency is function of the quantification of temperature change in time. Any bacterium that generates heat in a certain proportion of the control wells (to be determined in each case) is then considered a hit. The well co-ordinates will allow the identification of the corresponding enzyme

***Data processing:***

Data are processed in real time and stored in a database. The analysis is then carried to determine a specific enzyme combination that will allow, as a fingerprint, the fast identification of a pathogenic bacteria strain.

Bacteria contamination and strain identification can thus be processed in less than 4 hours by enzymatic profiling, using microcalorimetry in a microplate format. This process is likely to be automated using classical robotic equipment used in high throughput screening.

**Claims**

1. An array device for monitoring the effect of a physical or chemical stimulus on multiple small samples, said array device comprising a supporting substrate (14) with at least two array elements that are separated from each other by an isolation zone, said isolation zone being formed by at least part of said supporting substrate and being arranged to thermally isolate said array elements, each array element comprising:

   - a receiving zone (10) for retaining a sample, said receiving zone having a wall and being arranged to provide a contact between one of said multiple small samples and said physical or chemical stimulus, said receiving zone having a cross-section smaller than 10 mm, and
   - a differential heat detection means (13) arranged to perform a measurement of heat between said receiving zone and a reference,

   **characterized in that** said receiving zone of each of the array elements comprises a calibration resistor for calibrating the measurement of heat, the calibration resistor being situated in the wall of the receiving zone.

2. The array device according to claim 1, wherein the calibration resistor is designed to be able to produce dissipation heat in the same range as the produced biological power.

3. The array device according to any of the previous claims, wherein the reference is a neighbouring receiving zone.

4. The array device according to any of the previous claims, dimensioned as a standard 96, 384, 1536 or 6144-well microtiterplate.

5. The array device according to any of the previous claims, wherein said receiving zone furthermore comprises a thermosetting resistor for setting the array element at a pre-determined temperature.

6. The array device according to any of the previous claims, wherein said receiving zone comprises a bottom surface.

7. The array device according to claim 6, wherein said bottom surface comprises said substrate.

8. The array device according to claim 6 or 7, wherein said bottom surface is perforated.

9. The array device according to any of the previous claims, wherein said isolation zone comprises a bottom surface, formed by said supporting substrate, and an isolator comprised between the receiving zones.

10. The array device according to claim 9, wherein the isolator is air.

11. The array device according to any of the previous claims, wherein said differential heat detection means is selected from the group consisting of thermopile, thermistor, diode, IR detection means, CCD camera.

12. The array device according to any of the previous claims, wherein the receiving zone further comprises a detection means selected from the group consisting of a light addressable potentiometric sensor, a pair of interdigitated electrodes, a field effect transistor, a diode, a reference electrode and a working electrode.

13. The array device according to any of the previous claims, wherein said receiving zone is chemically modified to physically contain said sample and said chemical or physical stimulus within said receiving zone.

14. The array device according to any of the previous claims, wherein the receiving zones have a cross-section of between 10 $\mu$m and 9 mm.

15. The array device according to any of the previous claims, wherein the substrate is any of the group consisting of silicon, silicon dioxide, silicon nitride, silicon oxynitride, polysilicon, porous silicon, plastic, polymer, rubber, PVC, biodegradable polymer, glass, quartz, ceramics, aluminium oxide, agar, biological material.

16. The array device according to any of the previous claims, wherein the receiving zone is capable of handling volumes in the range of 0 ml to 100 ml, preferably in the range from 0 ml to 7 ml, more preferably in the range from 0 ml to 5 ml, most preferably in the range from 0.1 nl to 1 ml.

17. The array device according to any of the previous claims, wherein the receiving zone is a microvessel placed on top of, or hanging above the substrate.

18. The array device according to claim 17, wherein the microvessel is made of any of the group consisting of metal, steel, silicon, silicon oxide, silicon nitride, silicon oxynitride, polysilicon, porous silicon, plastic, polymer, rubber, PVC, biodegradable polymer, glass, quartz, ceramics, aluminium oxide, agar, biological material.

19. The array device according to any of the previous claims, wherein the substrate and/or the receiving zones comprise a membrane.

20. The array device according to any of the previous claims, wherein the array device furthermore comprises a lid covering it.

21. The array device according to any of the previous claims, the array device furthermore comprising a supply means, wherein the supply means has the same dimensions and layout as the device, except for the differential heat detection means which is omitted.

22. Calorimetric measuring method for measuring multiple samples, said method comprising the steps of:

   - providing an array device such as in any of the previous claims,
   - providing said samples in said receiving zones,
   - providing a chemical or physical stimulus to said samples, and
   - measuring the heat released by said samples.

23. Use of the array device of any of claims 1 to 21 for enzyme discovery.

24. Use of the array device of any of claims 1 to 21 for drug identification.

25. Use of the array device of any of claims 1 to 21 for agrochemical identification.


**Patentansprüche**

1. Arrayeinrichtung zum Überwachen der Wirkung eines physikalischen oder chemischen Reizes auf mehrere kleine Proben, welche ein Trägersubstrat (14) mit mindestens zwei Arrayelementen umfasst, die durch eine Isolationszone voneinander getrennt sind, wobei die Isolationszone von mindestens einem Teil des Trägersubstrats ausgebildet wird und dergestalt angeordnet ist, dass sie die Arrayelemente thermisch isoliert, und wobei die einzelnen Arrayelemente jeweils Folgendes aufweisen:

   - eine Aufnahmezone (10) zum Aufnehmen einer Probe, wobei die Aufnahmezone eine Wand aufweist und dergestalt angeordnet ist, dass sie einen Kontakt zwischen einer der mehreren kleinen Proben und dem physikalischen oder chemischen Reiz herstellt, und wobei die Aufnahmezone einen kleineren Querschnitt als 10 mm aufweist, und

- ein differentielles Wärmenachweismittel (13), das dazu eingerichtet ist, eine Messung der Wärme an der Aufnahmezone und an einer Referenz durchzuführen,

**dadurch gekennzeichnet, dass** die Aufnahmezonen der einzelnen Arrayelemente je einen Kalibrierwiderstand zum Kalibrieren der Wärmemessung aufweisen, welcher in der Wand der Aufnahmezone angeordnet ist.

2. Arrayeinrichtung nach Anspruch 1, wobei der Kalibrierwiderstand dazu ausgelegt ist, Dissipationswärme in demselben Bereich wie die erzeugte biologische Leistung zu erzeugen

3. Arrayeinrichtung nach einem der vorhergehenden Ansprüche, wobei die Referenz eine benachbarte Aufnahmezone ist.

4. Arrayeinrichtung nach einem der vorhergehenden Ansprüche, die als Standard-Mikrotiterplatte mit 96, 384, 1536 oder 6144 Wells dimensioniert ist.

5. Arrayeinrichtung nach einem der vorhergehenden Ansprüche, wobei die Aufnahmezone ferner einen Temperatureinstellungswiderstand zum Einstellen des Arrayelements auf eine vorbestimmte Temperatur aufweist.

6. Arrayeinrichtung nach einem der vorhergehenden Ansprüche, wobei die Aufnahmezone eine Bodenfläche aufweist.

7. Arrayeinrichtung nach Anspruch 6, wobei die Bodenfläche das Substrat umfasst.

8. Arrayeinrichtung nach Anspruch 6 oder 7, wobei die Bodenfläche perforiert ist.

9. Arrayeinrichtung nach einem der vorhergehenden Ansprüche, wobei die Isolationszone eine von dem Trägersubstrat gebildete Bodenfläche und einen zwischen den Aufnahmezonen eingeschlossenen Isolator umfasst.

10. Verfahren nach Anspruch 9, wobei der Isolator Luft ist.

11. Arrayeinrichtung nach einem der vorhergehenden Ansprüche, wobei das differentielle Wärmenachweismittel aus einer Thermosäule, einem Thermistor, einer Diode, einem IR-Nachweismittel und einer CCD-Kamera ausgewählt ist.

12. Arrayeinrichtung nach einem der vorhergehenden Ansprüche, wobei die Aufnahmezone ferner ein Nachweismittel aufweist, das aus einem Licht-adressierbaren potentiometrischen Sensor, einem Paar doppelkammförmig ineinandergreifender Elektroden, einem Feldeffekttransistor, einer Diode, einer Referenzelektrode und einer Arbeitselektrode ausgewählt ist.

13. Arrayeinrichtung nach einem der vorhergehenden Ansprüche, wobei die Aufnahmezone chemisch modifiziert ist, so dass sie die Probe und den chemischen oder physikalischen Reiz in der Aufnahmezone physikalisch enthält.

14. Arrayeinrichtung nach einem der vorhergehenden Ansprüche, wobei die Aufnahmezonen einen Querschnitt von zwischen 10 $\mu$m und 9 mm aufweisen.

15. Arrayeinrichtung nach einem der vorhergehenden Ansprüche, wobei es sich bei dem Substrat um Silicium, Siliciumdioxid, Siliciumnitrid, Siliciumoxynitrid, Polysilicium, poröses Silicium, Kunststoff, Polymer, Gummi, PVC, biologisch abbaubares Polymer, Glas, Quarz, Keramik, Aluminiumoxid, Agar oder biologisches Material handelt.

16. Arrayeinrichtung nach einem der vorhergehenden Ansprüche, wobei die Aufnahmezone dazu befähigt ist, Volumina im Bereich von 0 ml bis 100 ml, bevorzugt im Bereich von 0 ml bis 7 ml, besonders bevorzugt im Bereich von 0 ml bis 5 ml, ganz besonders bevorzugt im Bereich von 0,1 nl bis 1 ml aufzunehmen.

17. Arrayeinrichtung nach einem der vorhergehenden Ansprüche, wobei es sich bei der Aufnahmezone um ein Mikrogefäß handelt, das auf dem Substrat platziert ist oder über diesem hängt.

18. Arrayeinrichtung nach Anspruch 17, wobei das Mikrogefäß aus Metall, Stahl, Silicium, Siliciumoxid, Siliciumnitrid, Siliciumoxynitrid, Polysilicium, porösem Silicium, Kunststoff, Polymer, Gummi, PVC, biologisch abbaubarem Polymer, Glas, Quarz, Keramik, Aluminiumoxid, Agar oder biologischem Material hergestellt ist.

19. Arrayeinrichtung nach einem der vorhergehenden Ansprüche, wobei das Substrat und/oder die Aufnahmezonen eine Membran umfassen.

20. Arrayeinrichtung nach einem der vorhergehenden Ansprüche, wobei die Arrayeinrichtung ferner einen Deckel aufweist, der sie bedeckt.

21. Arrayeinrichtung nach einem der vorhergehenden Ansprüche, wobei die Arrayeinrichtung ferner ein Zuführungsmittel aufweist, wobei das Zuführungsmittel bis auf das fehlende differentielle Wärmenachweismittel dieselben Abmessungen und dieselbe Anordnung wie die Einrichtung aufweist.

22. Kalorimetrisches Messverfahren zum Messen mehrerer Proben, das folgende Schritte umfasst:

- Bereitstellen einer Arrayeinrichtung nach einem der vorherigen Ansprüche,
- Bereitstellen der Proben in den Aufnahmezonen,
- Bereitstellen eines chemischen oder physikalischen Reizes für die Proben und
- Messen der von den Proben entwickelten Wärme.

23. Benutzen der Arrayeinrichtung nach einem der Ansprüche 1 bis 21 für die Enzymforschung.

24. Benutzen der Arrayeinrichtung nach einem der Ansprüche 1 bis 21 zum Identifizieren von Arzneimitteln.

25. Benutzen der Arrayeinrichtung nach einem der Ansprüche 1 bis 21 zum Identifizieren von Agrochemikalien.

**Revendications**

1. Dispositif en réseaux pour le contrôle de l'effet d'un stimulus physique ou chimique sur de multiples petits échantillons, ledit dispositif en réseaux comprenant un substrat de support (14) avec au moins deux éléments en réseaux qui sont séparés l'un de l'autre par une zone d'isolation, ladite zone d'isolation étant formée par au moins ledit substrat de support et étant arrangée pour isoler thermiquement lesdits éléments en réseaux, chaque élément en réseaux comprenant :

- une zone réceptrice (10) pour conserver un échantillon, ladite zone réceptrice ayant une paroi et étant arrangée pour, fournir un contact entre l'un desdits multiples petits échantillons et ledit stimulus physique ou chimique, ladite zone réceptrice ayant une section transversale inférieure à 10 mm, et
- un moyen de détection de la chaleur différentielle (13) arrangée pour exécuter une mesure de chaleur entre ladite zone réceptrice et une référence,

**caractérisé en ce que** ladite zone réceptrice de chacun des éléments en réseaux comprend une résistance de calibration pour calibrer la mesure de la chaleur, la résistance de calibration étant située dans la paroi de la zone réceptrice.

2. Dispositif en réseaux selon la revendication 1, dans lequel la résistance de calibration est conçue pour être capable de produire une chaleur de dissipation dans le même domaine que la puissance biologique produite.

3. Dispositif en réseaux selon l'une quelconque des revendications précédentes, dans lequel la référence est une zone réceptrice voisine.

4. Dispositif en réseaux selon l'une quelconque des revendications précédentes, dimensionné comme une plaque de microtitration normale à 96, 384, 1 536 ou 6 144 puits.

5. Dispositif en réseaux selon l'une quelconque des revendications précédentes, dans lequel ladite zone réceptrice comprend en outre une résistance de réglage thermique pour le réglage des éléments en réseaux à une température prédéterminée.

**6.** Dispositif en réseaux selon l'une quelconque des revendications précédentes, dans lequel ladite zone réceptrice comprend une surface de fond.

**7.** Dispositif en réseaux selon la revendication 6, dans lequel la surface de fond comprend ledit substrat.

**8.** Dispositif en réseaux selon la revendication 6 ou 7, dans lequel la surface de fond est perforée.

**9.** Dispositif en réseaux selon l'une quelconque des revendications précédentes, dans lequel ladite zone d'isolation comprend une surface de fond, formée par ledit substrat de support, et un isolant compris entre les zones réceptrices.

**10.** Dispositif en réseaux selon la revendication 9, dans lequel l'isolant est l'air.

**11.** Dispositif en réseaux selon l'une quelconque des revendications précédentes, dans lequel ledit moyen de détection de chaleur différentielle est choisi dans le groupe constitué d'une thermopile, d'une thermistance, d'une diode, d'un moyen de détection IR, d'une caméra CCD.

**12.** Dispositif en réseaux selon l'une quelconque des revendications précédentes, dans lequel la zone réceptrice comprend en outre un moyen de détection choisi dans le groupe constitué d'un capteur potentiométrique adressable à la lumière, d'une paire d'électrodes interdigitées, d'un transistor à effet de champs, d'une diode, d'une électrode de référence et d'une électrode de travail.

**13.** Dispositif en réseaux selon l'une quelconque des revendications précédentes, dans lequel ladite zone réceptrice est chimiquement modifiée pour contenir physiquement ledit échantillon et ledit stimulus chimique ou physique à l'intérieur de ladite zone réceptrice.

**14.** Dispositif en réseaux selon l'une quelconque des revendications précédentes, dans lequel les zones réceptrices ont une section transversale comprise entre 10 $\mu$m et 9 mm.

**15.** Dispositif en réseaux selon l'une quelconque des revendications précédentes, dans lequel le substrat est l'un quelconque dans le groupe constitué du silicium, du dioxyde de silicium, du nitrure de silicium, de l'oxynitrure de silicium, du polysilicium, du silicium poreux, d'un plastique, d'un polymère, du caoutchouc, du PVC, d'un polymère biodégradable, du verre, du quartz, de la céramique, de l'oxyde d'aluminium, de l'agar, d'un matériau biologique.

**16.** Dispositif en réseaux selon l'une quelconque des revendications précédentes, dans lequel la zone réceptrice est capable de manier des volumes compris dans le domaine allant de 0 ml à 100 ml, de préférence dans le domaine allant de 0 ml à 7 ml, mieux dans le domaine allant de 0 ml à 5 ml, au mieux dans le domaine allant de 0,1 nl à 1 ml.

**17.** Dispositif en réseaux selon l'une quelconque des revendications précédentes, dans lequel la zone réceptrice est un microrécipient placé sur le dessus ou pendant au-dessus du substrat.

**18.** Dispositif en réseaux selon la revendication 17, dans lequel le microrécipient est fabriqué de l'un quelconque dans le groupe constitué d'un métal, de l'acier, du silicium, de l'oxyde de silicium, du nitrure de silicium, de l'oxynitrure de silicium, du polysilicium, du silicium poreux, d'un plastique, d'un polymère, du caoutchouc, du PVC, d'un polymère biodégradable, du verre, du quartz, de la céramique, de l'oxyde d'aluminium, de l'agar, d'un matériau biologique.

**19.** Dispositif en réseaux selon l'une quelconque des revendications précédentes, dans lequel le substrat et/ou les zones réceptrices comprennent une membrane.

**20.** Dispositif en réseaux selon l'une quelconque des revendications précédentes, dans lequel le dispositif en réseaux comprend en outre un couvercle le recouvrant.

**21.** Dispositif en réseaux selon l'une quelconque des revendications précédentes, le dispositif en réseaux comprenant en outre un moyen d'approvisionnement, dans lequel le moyen d'approvisionnement a les mêmes dimensions et plan que le dispositif, sauf pour le moyen de détection de la chaleur différentielle qui est omis.

**22.** Procédé de mesure calorimétrique pour mesurer des échantillons multiples, ledit procédé comprenant les étapes de

- fourniture d'un dispositif en réseaux tel que décrit selon l'une quelconque des revendications précédentes,

- fourniture desdits échantillons dans lesdites zones réceptrices,
- fourniture d'un stimulus chimique ou physique auxdits échantillons, et
- mesure de la chaleur libérée par lesdits échantillons.

23. Utilisation du dispositif en réseaux selon l'une quelconque des revendications 1 à 21 pour la découverte d'enzymes.

24. Utilisation du dispositif en réseaux selon l'une quelconque des revendications 1 à 21 pour l'identification de médicaments.

25. Utilisation du dispositif en réseaux selon l'une quelconque des revendications 1 à 21 pour l'identification agrochimique.

thermopile    sieve         full membrane

Fig. 1a

m=4 cell

full membrane

sieve with sensors

thermopile

rim with resistors

A                    A

capillary

cross section AA

sieve with sensors        thermopile

resistors

Fig. 1b

Fig. 2

m=4 cell          sieved lid          adhesive

sieve of sensor chip

Fig. 3

m=4 cell          air

Fig. 4

fluid delivery system

m=4 cell          sieve

Fig. 5

14    10

13

DHD          DHD

R    R          R    R

Fig. 6

Fig: 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6046056 A
- US 5104804 A
- WO 974573 A
- EP 0921391 A
- EP 0542422 A

### Non-patent literature cited in the description

- Nanotiterplates for screening and synthesis. **MAYER G. et al.** Biomethods. Birkhaeuser, 1999, 75-128